# EUROPEAN PATENT APPLICATION

(11) **EP 4 722 255 A1**
(43) Date of publication of application: **08.04.2026**
(21) Application number: 24813852.1
(22) Date of filing: 25.03.2024
(51) Int. Cl.: C07K 19/00, C12N 15/62, C12N 15/867, C12N 5/10, A61K 39/00, A61P 35/00

(54) **CHIMERIC ANTIGEN RECEPTOR AND USE THEREOF**

(30) Priority: 31.05.2023 CN 202310639033
(71) Applicant: Shenzhen Pregene Biopharma Co. Ltd., Shenzhen, Guangdong 518118 (CN)
(72) Inventor: ZHANG, Jishuai, Shenzhen, Guangdong 518118 (CN); LI, Hongjian, Shenzhen, Guangdong 518118 (CN); SU, Hongchang, Shenzhen, Guangdong 518118 (CN); BIN, Zhenlan, Shenzhen, Guangdong 518118 (CN); LUO, Yanping, Shenzhen, Guangdong 518118 (CN); XIANG, Bin, Shenzhen, Guangdong 518118 (CN); WANG, Ling, Shenzhen, Guangdong 518118 (CN); ZHANG, Bingxiang, Shenzhen, Guangdong 518118 (CN)
(74) Representative: Winter, Brandl - Partnerschaft mbB
(86) International application number: PCT/CN2024/083573
(87) International publication number: WO 2024/244646

(57) **Abstract**

The invention discloses a chimeric antigen receptor and relates to the technical field of biomedicine. The chimeric antigen receptor includes a BCMA binding domain and a non-antigen binding domain, wherein the non-antigen binding domain includes an extracellular domain, a transmembrane domain and an intracellular co-stimulatory domain of CD28 or a functional variant thereof, and a signaling domain of CD3ζ or a functional variant thereof; and the enhancer is selected from IL-2, IL-3, IL-4, IL-6, IL-7, IL-8, IL-10, IL-11, IL-12, IL-15, IL-17, IL-18, IL-21, IL-23, receptors or functional variants thereof or their combination. The present application also relates to a CAR-NK cell including the chimeric antigen receptor. The tumor-targeting CAR-NK cell are more effective in killing tumor cells and can be used as a CAR-NK cell drug for the treatment of tumors.

## Description

The present application claims the priority of China patent application with application number of 202210620008.2 filed on June 2, 2022, named "Chimeric antigen receptors and uses thereof", and the priority of China patent application with application number of 202310639033.X filed in China Patent Office on May 31, 2023, named "Chimeric antigen receptors and uses thereof". The entire contents of which are incorporated by reference in this application.

### Technical field

The present application belongs to the technical field of biomedicine, and in particular to a chimeric antigen receptor and uses thereof.

### Background Art

The two main methods of immunotherapy are immune checkpoint inhibitors, such as anti-PD1/PDL1 antibodies and anti-CTLA4 antibodies, and chimeric antigen receptor T cells (CAR-T). The CAR-T treatment process is to separate T cells from human peripheral blood, perform CAR gene modification in vitro, expand and culture them to a certain number, and then reinfuse the patient. In particular, the design and development of CAR genes is CAR-T's core technology. By transferring the designed CAR gene into the genome of T cells, T cells can express the corresponding CAR molecules. CAR molecules will enable T cells to directly recognize tumor cells through the single-chain variable region fragment scFv without MHC restriction, and simultaneously obtain the first stimulatory signal and co-stimulatory signal and be "One-Key activated". CAR-T can proliferate in vivo and serve as a "living drug" and form a continuous killing force. CAR-T can also release cytokines in vivo to recruit and activate more immune cells to collaboratively kill tumor cells. Although the existing CAR-T drug technology is mature and has significant efficacy, there are still many problems. For example, patients need to wait for the production of pharmaceuticals (not in stock), during which the tumor may progress that causes increasing the risk of treatment or losing the opportunity for treatment; and more, preparation failure may result in the drug being unavailable; furthermore, the issue of safety and high price is also in existence. To solve the current main drawback of autologous CAR-T cell drugs (not in stock and high price), universal cell drug usually in stock should be developed, such as universal CAR-T, universal CAR-NK, etc. Spot CAR T or CAR NK could be prepared dozens in one batch, using allogeneic T cells or NK cells, or using T cells or NK cells differentiated from iPS cells by inducing. Compared with autologous CAR-T cell drugs, the universal CAR-T, or CAR-NK reduces the cost and lower the price. CAR-NK technology is an immune cell therapy strategy accompanied by autologous CAR-T technology. Initially, in order to achieve the purpose of spot cell drugs, CAR-modified NK cells mainly used NK cell line NK-92 (which can be expanded infinitely). However, because of the risk of tumor formation, NK-92 should be irradiated before use. The CAR-NK-92 cannot be amplified in vivo, and the reported clinical efficacy is not satisfactory. The CAR structure used by NK cells requires special design. Currently, new CAR-NK therapy is still needed to meet the needs of patients.

### Summary

The purpose of the application is to provide a chimeric antigen receptor, the CAR-NK targeting BCMA has a better killing effect on tumor cells.

In order to achieve the above-mentioned purpose of the application, the technical solution of the present application is as follows:
In one aspect, the present application provides a fusion polypeptide comprising a chimeric antigen receptor (CAR) according to the present application and an enhancer capable of enhancing one or more activities of immune effector cells, wherein the chimeric antigen receptor includes a BCMA binding domain and a non-antigen binding domain, wherein the non-antigen binding domain includes the extracellular domain, the transmembrane domain and the intracellular co-stimulatory domain of CD28 or a functional variant thereof, and the signaling domain of CD3ζ or a functional variant thereof; and the enhancer is selected from IL-2, IL-3, IL-4, IL-6, IL-7, IL-8, IL-10, IL-11, IL-12, IL-15, IL-17, IL-18, IL-21, IL-23, one or more of receptors or functional variants thereof.

In certain embodiments, the BCMA-binding domain includes heavy chain complementary determining region 1 (HCDR1), heavy chain complementary determining region 2 (HCDR2) and heavy chain complementary determining region 3 (HCDR3), wherein the HCDR1 includes an amino acid sequence shown in SEQ ID NO: 2, the HCDR2 includes an amino acid sequence shown in SEQ ID NO: 3, and the HCDR3 includes an amino acid sequence shown in SEQ ID NO: 4.

In certain embodiments, the HCDR1 includes an amino acid sequence that is at least about 50% identical to the amino acid sequence shown in SEQ ID NO:2, the HCDR2 includes an amino acid sequence that is at least about 50% identical to the amino acid sequence shown in SEQ ID NO:3, and the HCDR3 includes an amino acid sequence that is at least about 50% identical to the amino acid sequence shown in SEQ ID NO:4.

In certain embodiments, the BCMA-binding domain includes a single domain antibody.

In certain embodiments, the BCMA-binding domain includes an amino acid sequence shown in SEQ ID NO:8; or an amino acid sequence that is at least about 50% identical to the amino acid sequence shown in SEQ ID NO:8.

In certain embodiments, the extracellular domain, transmembrane domain and intracellular co-stimulatory domain of CD28 include an amino acid sequence shown in SEQ ID NO:9; or an amino acid sequence that is at least about 50% identical to the amino acid sequence shown in SEQ ID NO:9.

In certain embodiments, the signaling domain of CD3ζ includes an amino acid sequence shown in SEQ ID NO:5; or an amino acid sequence that is at least about 50% identical to the amino acid sequence shown in SEQ ID NO:5.

In certain embodiments, the non-antigen binding domain includes an amino acid sequence shown in SEQ ID NO:10; or an amino acid sequence that is at least about 50% identical to the amino acid sequence shown in SEQ ID NO:10.

In certain embodiments, the antigen binding domain specifically binds to a tumor antigen.

In certain embodiments, the tumor antigen is associated with a non-solid tumor.

In certain embodiments, the non-solid tumor is selected from the group consisting of lymphocytic neoplasms, Hodgkin's lymphoma, chronic and acute lymphocytic leukemia, chronic myeloid leukemia, acute myeloid leukemia, and multiple myeloma.

In certain embodiments, the tumor antigen is associated with a solid tumor.

In certain embodiments, the solid tumor is selected from the group consisting of liver cancer, gastric cancer, small cell lung cancer, non-small cell lung cancer, esophageal cancer, pancreatic cancer, colorectal cancer, breast cancer, prostate cancer, and ovarian cancer.

In certain embodiments, the tumor antigen includes a tumor antigen selected from the group consisting of CD5, CD7, CD38, CS1, BAFFR, TACI, CD19, CD20, CD22, BCMA, GPRC5D, Mesothelin, DLL1, GPC3, EGFR, PSMA, PSCA, Claudin18.2, HER2 and CD70.

In certain embodiments, the chimeric antigen receptor further includes a signal peptide fragment, and a C-terminal of the signal peptide fragment is linked with a N-terminal of the BCMA binding domain; the signal peptide fragment includes an amino acid sequence shown in SEQ ID NO:1; or an amino acid sequence that is at least about 50% identical to the amino acid sequence shown in SEQ ID NO:1.

In certain embodiments, the chimeric antigen receptor includes an amino acid sequence shown in SEQ ID NO:11 or an amino acid sequence shown in SEQ ID NO:12.

In certain embodiments, the enhancer includes cytokines and/or cytokine receptors.

In certain embodiments, the enhancer includes IL-15 or its functional variant, or includes a chimeric cytokine receptor of IL-15 receptor (IL15R).

In certain embodiments, the enhancer includes an amino acid sequence shown in SEQ ID NO:7; or an amino acid sequence that is at least about 50% identical to the amino acid sequence shown in SEQ ID NO:7.

In certain embodiments, the chimeric antigen receptor and the enhancer are linked by a linker. In certain embodiments, the linker includes a cleavable linker.

In certain embodiments, the linker includes a self-cleaving peptide.

In certain embodiments, the self-cleaving peptide includes P2A, F2A, E2A or T2A.

In certain embodiments, the self-cleaving peptide is T2A, the N-terminal of the chimeric antigen receptor and the C-terminal of the enhancer can be linked through T2A, and have the following structure: IL-15-T2A-CD8SP-anti-BCMA sdAb-CD28(hinge/TM/Intracellular)-CD3ζ; or, the C-terminal of the chimeric antigen receptor and the N-terminal of the enhancer can be linked through T2A, and have the following structure: CD8SP-anti-BCMA sdAb-CD28(hinge/TM/Intracellular)-CD3ζ-T2A-IL-15.

In certain embodiments, the linker includes an amino acid sequence shown in SEQ ID NO:6; or an amino acid sequence that is at least about 50% identical to the amino acid sequence shown in SEQ ID NO:6.

In certain embodiments, the fusion polypeptide includes an amino acid sequence shown in SEQ ID NO:13; or an amino acid sequence that is at least about 50% identical to the amino acid sequence shown in SEQ ID NO:13.

In another aspect, the application provides an isolated nucleic acid molecule, which encodes the fusion polypeptide of the application.

In another aspect, the application provides one or more isolated nucleic acid molecules, which includes a nucleotide sequence encoding a chimeric antigen receptor, wherein the chimeric antigen receptor includes a BCMA binding domain and a non-antigen binding domain, wherein the non-antigen binding domain includes an extracellular domain, a transmembrane domain and an intracellular co-stimulatory domain of CD28 or a functional variant thereof, and a signaling domain of CD3ζ or a functional variant thereof; and a nucleotide sequence encoding an enhancer capable of enhancing one or more activities of immune effector cells.

In certain embodiments, the extracellular domain, transmembrane domain and intracellular co-stimulatory domain of CD28 include an amino acid sequence shown in SEQ ID NO:9.

In certain embodiments, the nucleic acid molecule may include a nucleotide sequence described in SEQ ID NO:17.

In certain embodiments, the signal transduction domain of CD3ζ includes an amino acid sequence shown in SEQ ID NO:5.

In certain embodiments, the nucleic acid molecule may include a nucleotide sequence described in SEQ ID NO:18.

In certain embodiments, the non-antigen binding domain includes an amino acid sequence shown in SEQ ID NO:10.

In certain embodiments, the antigen binding domain specifically binds to a tumor antigen.

In certain embodiments, the tumor antigen is associated with a non-solid tumor.

In certain embodiments, the non-solid tumor is selected from the group consisting of B-lymphocytic neoplasms, Hodgkin's lymphoma, chronic myeloid leukemia, and acute myeloid leukemia.

In certain embodiments, the tumor antigen is associated with a solid tumor.

In certain embodiments, the solid tumor is selected from the group consisting of liver cancer, gastric cancer, lung cancer, breast cancer, and non-small cell lung cancer.

In certain embodiments, the tumor antigen includes a tumor antigen selected from the group consisting of CD5, CD19, CD20 and BCMA.

In certain embodiments, the BCMA-binding domain includes heavy chain complementary determining region 1 (HCDR1), heavy chain complementary determining region 2 (HCDR2) and heavy chain complementary determining region 3 (HCDR3), wherein the HCDR1 includes an amino acid sequence shown in SEQ ID NO: 2, the HCDR2 includes an amino acid sequence shown in SEQ ID NO: 3, and the HCDR3 includes an amino acid sequence shown in SEQ ID NO: 4.

In certain embodiments, the BCMA-binding domain includes a single domain antibody.

In certain embodiments, the BCMA binding domain includes an amino acid sequence shown in SEQ ID NO:8.

In certain embodiments, the nucleic acid molecule may include a nucleotide sequence described in SEQ ID NO:16.

In certain embodiments, the chimeric antigen receptor includes an amino acid sequence shown in SEQ ID NO:11.

In certain embodiments, the chimeric antigen receptor further includes a signal peptide fragment, wherein a C-terminus of the signal peptide fragment is linked to a N-terminus of the antigen binding domain.

In certain embodiments, the signal peptide fragment includes an amino acid sequence shown in SEQ ID NO:1.

In certain embodiments, the chimeric antigen receptor includes an amino acid sequence shown in SEQ ID NO:12.

In certain embodiments, the nucleic acid molecule may include a nucleotide sequence of SEQ ID NO:21; or a nucleotide molecule that is at least about 50% identical to the nucleotide sequence shown in SEQ ID NO:21.

In certain embodiments, the nucleotide sequence encoding the chimeric antigen receptor includes the nucleotide sequence shown in SEQ ID NO:22.

In certain embodiments, the nucleotide sequence encoding the chimeric antigen receptor includes a nucleotide sequence that is at least about 50% identical to the nucleotide sequence shown in SEQ ID NO:22.

In certain embodiments, the enhancer includes cytokines and/or cytokine receptors.

In certain embodiments, the enhancer is selected from IL-2, IL-3, IL-4, IL-6, IL-7, IL-8, IL-10, IL-11, IL-12, IL-15, IL-17, IL-18, IL-21, IL-23, its receptors, its functional variants and combinations thereof.

In certain embodiments, the enhancer includes IL-15 or its functional variant, or includes a chimeric cytokine receptor of IL-15 receptor (IL15R).

In certain embodiments, the enhancer includes an amino acid sequence shown in SEQ ID NO:7.

In certain embodiments, the nucleotide sequence encoding the enhancer is shown in SEQ ID NO:20.

In certain embodiments, the chimeric antigen receptor and the enhancer are linked by a linker. In certain embodiments, the linker includes a cleavable linker.

In certain embodiments, the linker includes a self-cleaving peptide.

In certain embodiments, the self-cleaving peptide includes P2A, F2A, E2A or T2A.

In certain embodiments, the linker includes an amino acid sequence shown in SEQ ID NO:6. In certain embodiments, the nucleic acid encoding the linker includes the nucleotide sequence shown in SEQ ID NO:19.

In certain embodiments, the nucleic acid molecule includes a nucleotide sequence shown in SEQ ID NO:23.

In another aspect, the application provides isolated one or more constructs, which include the nucleic acid molecule or the isolated one or more nucleic acid molecules according to the application.

In certain embodiments, the isolated one or more nucleic acid molecules include a first nucleic acid molecule and a second nucleic acid molecule, wherein the first nucleic acid molecule includes a nucleotide sequence encoding a chimeric antigen receptor, wherein the chimeric antigen receptor includes a BCMA binding domain and a non-antigen binding domain, wherein the non-antigen binding domain includes an extracellular domain, a transmembrane domain and an intracellular co-stimulatory domain of CD28 or a functional variant thereof, and a signaling domain of CD3ζ or a functional variant thereof.

In certain embodiments, the extracellular domain, transmembrane domain and intracellular co-stimulatory domain of CD28 include an amino acid sequence shown in SEQ ID NO:9.

In certain embodiments, the first nucleic acid molecule may include a nucleotide sequence shown in SEQ ID NO:17.

In certain embodiments, the signal transduction domain of CD3ζ includes an amino acid sequence shown in SEQ ID NO:5.

In certain embodiments, the first nucleic acid molecule may include a nucleotide sequence shown in SEQ ID NO:18.

In certain embodiments, the non-antigen binding domain includes an amino acid sequence shown in SEQ ID NO:10.

In certain embodiments, the BCMA-binding domain includes heavy chain complementary determining region 1 (HCDR1), heavy chain complementary determining region 2 (HCDR2) and heavy chain complementary determining region 3 (HCDR3), wherein the HCDR1 includes an amino acid sequence shown in SEQ ID NO: 2, the HCDR2 includes an amino acid sequence shown in SEQ ID NO: 3, and the HCDR3 includes an amino acid sequence shown in SEQ ID NO: 4; the second nucleic acid molecule includes a nucleotide sequence encoding an enhancer capable of enhancing one or more activities of immune effector cells.

In certain embodiments, the BCMA-binding domain includes a single domain antibody.

In certain embodiments, the BCMA binding domain includes an amino acid sequence shown in SEQ ID NO:8.

In certain embodiments, the first nucleic acid molecule may include a nucleotide sequence shown in SEQ ID NO:16.

In certain embodiments, the first nucleic acid molecule may include a nucleotide sequence shown in SEQ ID NO:21.

In certain embodiments, the chimeric antigen receptor includes an amino acid sequence shown in SEQ ID NO:11.

In certain embodiments, the chimeric antigen receptor further includes a signal peptide fragment, wherein a C-terminus of the signal peptide fragment is linked to a N-terminus of the antigen binding domain.

In certain embodiments, the signal peptide fragment includes an amino acid sequence shown in SEQ ID NO:1.

In certain embodiments, the chimeric antigen receptor includes an amino acid sequence shown in SEQ ID NO:12.

In certain embodiments, the first nucleic acid molecule includes a nucleotide sequence shown in SEQ ID NO:22.

In certain embodiments, the enhancer includes cytokines and/or cytokine receptors.

In certain embodiments, the enhancer is selected from IL-2, IL-3, IL-4, IL-6, IL-7, IL-8, IL-10, IL-11, IL-12, IL-15, IL-17, IL-18, IL-21, IL-23, its receptors, its functional variants or combinations thereof.

In certain embodiments, the enhancer includes IL-15 or its functional variant, or includes a chimeric cytokine receptor of IL-15 receptor (IL15R).

In certain embodiments, the enhancer includes an amino acid sequence shown in SEQ ID NO:7. In certain embodiments, the second nucleic acid molecule includes a nucleotide sequence shown in SEQ ID NO:20.

In certain embodiments, the first nucleic acid molecule and the second nucleic acid molecule are located in the same construct.

In certain embodiments, the chimeric antigen receptor and the enhancer are linked by a linker. In certain embodiments, the linker includes a cleavable linker.

In certain embodiments, the linker includes a self-cleaving peptide.

In certain embodiments, the self-cleaving peptide includes P2A, F2A, E2A or T2A.

In certain embodiments, the linker includes an amino acid sequence shown in SEQ ID NO:6. In certain embodiments, the nucleic acid molecule includes a nucleotide sequence shown in SEQ ID NO:23.

In certain embodiments, the first nucleic acid molecule and the second nucleic acid molecule are located in separate constructs.

The constructs of the application may be present in a vector, or may be integrated into the genome.

In certain embodiments, the vector is selected from DNA vectors, an RNA vectors, plasmids, lentiviral vectors, adenoviral vectors and retroviral vectors.

In another aspect, the application provides a cell, which includes the nucleic acid molecule of the application, or the isolated one or more nucleic acid molecules described above or the construct of the application, and/or expresses the chimeric antigen receptor of the application or the fusion polypeptide of the application.

In certain embodiments, the cell includes immune effector cells.

In certain embodiments, the immune effector cell includes T cells, B cells, natural killer cells (NK cells), macrophages, NKT cells, monocytes, dendritic cells, granulocytes, lymphocytes, leukocytes and/or peripheral blood mononuclear cells.

In certain embodiments, the immune effector cell includes engineered immune effector cells. In certain embodiments, the engineered immune effector cell includes CAR-T cells or CAR-NK cells.

In certain embodiments, the cell expresses:
(i) an enhancer capable of enhancing one or more activities of immune effector cells; (ii) a chimeric antigen receptor, which includes a BCMA binding domain and a non-antigen binding domain, wherein the non-antigen binding domain includes an extracellular domain, a transmembrane domain and an intracellular co-stimulatory domain of CD28 or a functional variant thereof, and a signaling domain of CD3ζ or a functional variant thereof.

The cell can be a CAR-NK cell, which expresses: (i) an enhancer capable of enhancing one or more activities of the CAR-NK cell; (ii) a chimeric antigen receptor, which includes a BCMA binding domain and a non-antigen binding domain, wherein the non-antigen binding domain includes an extracellular domain, a transmembrane domain and an intracellular co-stimulatory domain of CD28 or a functional variant thereof, and a signaling domain of CD3ζ or a functional variant thereof.

In certain embodiments, the cell expresses: (i) IL-15 or a functional variant thereof; (ii) a chimeric antigen receptor, which includes a BCMA binding domain and a non-antigen binding domain, wherein the non-antigen binding domain includes an extracellular domain, a transmembrane domain and an intracellular co-stimulatory domain of CD28 or a functional variant thereof, and a signaling domain of CD3ζ or a functional variant thereof.

The cell can be a CAR-NK cell, which expresses: (i) IL-15 or a functional variant thereof; (ii) a chimeric antigen receptor, which includes a BCMA binding domain and a non-antigen binding domain, wherein the non-antigen binding domain includes an extracellular domain, a transmembrane domain and an intracellular co-stimulatory domain of CD28 or a functional variant thereof, and a signaling domain of CD3ζ or a functional variant thereof.

The application provides an immune effector cell expressing IL-15, which expresses IL-15 and a chimeric antigen receptor, the chimeric antigen receptor includes a BCMA binding domain and a non-antigen binding domain, wherein the non-antigen binding domain includes an extracellular domain, a transmembrane domain and an intracellular co-stimulatory domain of CD28 or a functional variant thereof, and a signaling domain of CD3ζ or a functional variant thereof.

In certain embodiments, wherein the IL-15 or its functional variant is expressed as a membrane-bound polypeptide and/or a secreted polypeptide. In some embodiments, the IL-15 or its functional variants can be expressed as a secreted polypeptide. In some other embodiments, the IL-15 or its functional variants are fused to a transmembrane protein. "Functional variants" of IL-15 include a part of IL-15 that maintains one or more functions of full-length or mature IL-15. IL-15 or its functional variants can be expressed by one or more NK cells in a variety of ways. IL-15 can be expressed within NK cells and secreted by NK cells and/or can be linked directly or indirectly using any of a variety of linkers known in the art. In some specific embodiments, IL-15 can be linked to all or part of a transmembrane protein. In one aspect, NK cells express fusion proteins including IL-15 fused to transmembrane proteins or transmembrane domains thereof. In certain embodiments, the cell includes (i) a first nucleic acid molecule, which includes nucleotide sequence encoding a chimeric antigen receptor, wherein the chimeric antigen receptor includes a BCMA binding domain and a non-antigen binding domain, wherein the non-antigen binding domain includes an extracellular domain, a transmembrane domain and an intracellular co-stimulatory domain of CD28 or a functional variant thereof, and a signaling domain of CD3ζ or a functional variant thereof; and (ii) a second nucleic acid molecule, which includes a nucleotide sequence encoding an enhancer capable of enhancing one or more activities of the engineered immune effector cells. In certain embodiments, the cell includes (i) a first nucleic acid molecule, which includes nucleotide sequence encoding a chimeric antigen receptor, wherein the chimeric antigen receptor includes a BCMA binding domain and a non-antigen binding domain, wherein the non-antigen binding domain includes an extracellular domain, a transmembrane domain and an intracellular co-stimulatory domain of CD28 or a functional variant thereof, and a signaling domain of CD3ζ or a functional variant thereof; and (ii) a second nucleic acid molecule, which includes a nucleotide sequence encoding an exogenous cytokine IL-15 or a functional variant thereof or a nucleotide sequence encoding a chimeric cytokine receptor including an IL-15 receptor.

In another aspect, the application provides a method for modifying a cell, the method includes: introducing a first nucleic acid molecule and a second nucleic acid molecule into the cell, wherein:
(i) the first nucleic acid molecule encodes a chimeric antigen receptor, the chimeric antigen receptor includes a BCMA binding domain and a non-antigen binding domain, wherein the non-antigen binding domain includes an extracellular domain, a transmembrane domain and an intracellular co-stimulatory domain of CD28 or a functional variant thereof, and a signaling domain of CD3ζ or a functional variant thereof; and (ii) the second nucleic acid molecule encodes a nucleotide sequence of an enhancer capable of enhancing one or more activities of the engineered immune effector cells.

In another aspect, the application provides a pharmaceutical composition, which includes the fusion polypeptide of the application, the nucleic acid molecule of the application, the isolated one or more constructs of the application or the cell of the application, and optionally a pharmaceutically acceptable carrier.

In another aspect, the application provides the use of the fusion polypeptide, the nucleic acid molecule, the isolated one or more nucleic acid molecules, the isolated one or more constructs, or the cells of the application for preparing drugs, wherein the drugs are used for treating tumors. The beneficial effects of the application are in that:
The application provides a chimeric antigen receptor. This CAR molecule uses a single domain antibody to target and recognize BCMA, and the extracellular hinge region, transmembrane region and cytoplasmic region use the extracellular region, transmembrane region and cytoplasmic region of the CD28 molecule to link to the intracellular domain of CD3ζ, and link to the wild-type IL-15 cytokines through T2A. Cell experiments and animal experiments show that the CAR-NK targeting BCMA has a good effect of killing tumor cells, which can be further developed as a CAR-NK cell drug for the treatment of multiple myeloma. Whether CAR-NK prepared by cytokine amplification or CAR-NK prepared by incubating and activating K562 feeder cells modified by expressing mbIL-21, CD137L and CD86 genes, CAR-NK targeting BCMA has good killing effect. Animal experiments show that CAR-NK prepared by activation of feeder cells can effectively inhibit the proliferation of myeloma cell line MM.1S

### Description of the drawings

The specific features of the application to which the application relate are shown in the appended claims. The features and advantages of the application to which the present application relates can be better understood by referring to the exemplary embodiments described in detail-below and the accompanying drawings. A brief description of the accompanying drawings is as follows:

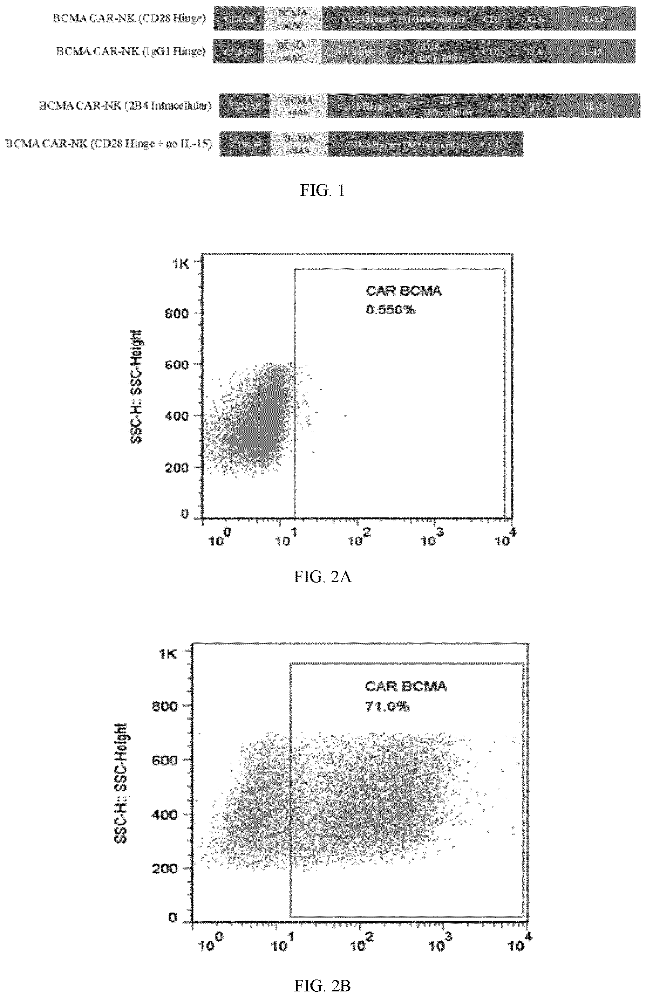

FIG. 1 shows a schematic structural diagram of an anti-BCMA CAR-NK according to the application; wherein CD8SP represents the signal peptide of CD8 molecule; BCMA sdAb represents the single domain antibody sequence targeting BCMA; CD28Hinge+TM+Intracellular represents the extracellular region, transmembrane region and cytoplasmic region sequence of CD28 molecule; CD3ζ represents the cytoplasmic region sequence of CD3ζ molecule; T2A represents the self-cleavage sequence; IL-15 represents the IL-15 sequence; IgG1hinge represents the hinge region of IgG1; 2B4 Intracellular represents the 2B4 cytoplasmic region sequence.
FIG. 2A - FIG. 2B shows the positive rate detection results of the anti-BCMA CAR-NK (CD 28hinge) cells prepared in Embodiment 2 of the application.
FIG. 3 shows the killing rate of anti-BCMA CAR-NK (CD28 hinge) cells prepared in Embodiment 2 of the application in killing multiple myeloma cell line MM. 1S in vitro.
FIG. 4A - FIG. 4B shows the positive rate detection results of the anti-BCMA CAR-NK (CD28 hinge) cells prepared in Embodiment 3 of the application.
FIG. 5 shows the killing rate of anti-BCMA CAR-NK (CD28 hinge) cells prepared in Embodiment 3 of the application in killing multiple myeloma cell line MM.1S in vitro.
FIG. 6 shows the comparison of the killing rates of anti-BCMA CAR-NK (CD28 hinge) cells and anti-BCMA CAR-NK (IgG1 hinge) cells prepared in Embodiment 3 of the application in killing multiple myeloma cell line MM.1S in vitro.
FIG. 7 shows the comparison of the killing rates of anti-BCMA CAR-NK (CD28 hinge) cells and anti-BCMA CAR-NK (2B4 Intracelluar) cells prepared in Embodiment 3 of the application in killing multiple myeloma cell line MM.1S in vitro.
FIG. 8 shows the comparison of the killing rates of anti-BCMA CAR-NK (CD28 hinge) cells and anti-BCMA CAR-NK (CD28 hinge+no IL-15) cells prepared in Embodiment 3 of the application in killing multiple myeloma cell line MM.1S in vitro.
FIG. 9 shows the expression results of cytokine IFN-γ in anti-BCMA CAR-NK (CD28 hinge) cells and anti-BCMA CAR-NK (IgG1 hinge) cells prepared in Embodiment 3 of the application.
FIG. 10 shows the results of killing tumor cells with different levels of BCMA expression in vitro by anti-BCMA CAR-NK (CD28 hinge) cells prepared in Embodiment 3 of the application.
FIG. 11 shows the animal experimental effect of MM.1S of anti-BCMA CAR-NK (CD28 hinge) cell prepared in Embodiment 3 of the application.

### Detailed description

In order to make the technical means, creative features, objectives and effects achieved by the application easy to understand, the application is further explained below in conjunction with specific embodiments, but the following embodiments are only preferred embodiments of the application, not all. Based on the embodiments, all other embodiments obtained by those skilled in the art without making any creative work shall fall within the scope of protection of the application. In the following embodiments, unless otherwise specified, the operating methods used are all conventional operating methods, the equipment used is all conventional equipment, and the equipment and materials used in each embodiment are the same.

### Definition of terms

In the application, the term "BCMA" generally refers to a cell maturation antigen, which is a member of the tumor necrosis factor receptor superfamily.

In the application, the term "BCMA binding domain" generally refers to a humanized anti-BCMA antibody or an antigen-binding fragment thereof that can specifically bind to a BCMA polypeptide expressed on B cells.

In the application, the term "antigen-binding fragment" (also referred to herein as "targeting moiety" or "antigen-binding moiety") generally refers to a part of an antibody molecule that includes amino acids responsible for the specific binding between the antibody and the antigen. The part of an antigen that is specifically recognized and bound by an antibody is called an "epitope" as described above. An antigen binding domain may typically include an antibody light chain variable region (VL) and an antibody heavy chain variable region (VH); however, it does not necessarily include both.

In the application, the term "complementary determining region" (CDR) generally refers to the complementarity determining region within the variable region of an antigen binding fragment. In the application, the heavy chain variable region has three CDRs, which are named HCDR1, HCDR2 and HCDR3 for each variable region.

In the application, the term "FR" generally refers to the more highly conserved parts of antibody variable domains, which are called the framework regions.

In the application, the term "single domain antibody (sdAb)" or "VHH" generally refers to a type of antibody that lacks the antibody light chain and only has the heavy chain variable region.

In the application, the term "chimeric antigen receptor" or "CAR" generally refers to a group of polypeptides, usually two in the simplest embodiment, which when in an immune effector cell, provide the cell with specificity for a target cell (usually a cancer cell) and generate an intracellular signal. In some embodiments, the CAR includes at least one extracellular antigen binding domain (such as a VHH, scFv or part thereof), a transmembrane domain and a cytoplasmic signaling domain (herein also referred to an "intracellular signaling domain"), which includes a functional signaling domain derived from a stimulatory molecule and/or a co-stimulatory molecule as defined below.

In the application, the term "functional variant" generally refers to an amino acid sequence having substantially the same function as that of the variant and having at least 85% sequence identity therewith.

In the application, the term "isolated nucleic acid molecule" generally refers to isolated forms of nucleotides, deoxyribonucleotides or ribonucleotides of any length or their analogues isolated from their natural environment or artificially synthesized.

In the application, the term "construct" generally refers to a nucleic acid molecule capable of self-replication in a suitable host, which transfers an inserted nucleic acid molecule into a host cell and/or between host cells.

In the application, the term "immune effector cell" generally refers to an immune cell that participates in an immune response and performs effector functions.

In the application, the term "natural killer cell" ("NK cell") generally refers to a type of cytotoxic lymphocyte of the immune system.

In the application, the term "interleukin-15" generally refers to cytokines that regulate T and NK cell activation and proliferation. In one aspect, IL-15 is wild-type IL-15.

In the application, the term "expression" generally refers to the transcription and/or translation of a specific nucleotide sequence.

In the application, the terms "tumor" and "cancer" are used interchangeably and generally refer to a disease characterized by the rapid and uncontrolled growth of abnormal cells. Examples of cancer herein include, but are not limited to breast cancer, prostate cancer, ovarian cancer, cervical cancer, skin cancer, pancreatic cancer, colorectal cancer, kidney cancer, liver cancer, brain cancer, lymphoma, leukemia, lung cancer, etc. It includes pre-malignant as well as malignant cancers and tumors, and covers both solid and non-solid tumors.

In the application, the term "administering" generally refers to delivering protein to a human or animal in need thereof by any route known in the art. Pharmaceutical carriers and preparations or compositions are also well known in the art.

As used herein, the term "pharmaceutically acceptable carrier" or "pharmaceutically acceptable excipient" refers to any and all solvents, dispersion media, preservatives, antioxidants, coatings, isotonic and absorption delaying agents, surfactants, fillers, disintegrants, binders, diluents and lubricants that are compatible with drug administration, glidants, pH adjusters, buffers, enhancers, wetting agents, solubilizing agents, surfactants, antioxidants, etc.

In the application, the term "effective amount" or "effective dose" generally refers to an amount sufficient to achieve or at least partially achieve the desired effect. A "therapeutically effective amount" or "therapeutically effective dose" of a drug or therapeutic agent is generally any amount of the drug that promotes regression of a disease when used alone or in combination with another therapeutic agent.

In the application, the term "include" generally means including, encompassing, containing or having.

In the application, the term "subject" generally refers to a human or a non-human animal.

### Embodiment 1:

### 1.1 Construction of chimeric antigen receptor

As shown in Fig. 1, using the anti-BCMA single domain antibody sequence (SEQ ID NO: 8), combining the extracellular region, transmembrane region and cytoplasmic region of CD28 molecule as the extracellular hinge region, transmembrane region and cytoplasmic region (SEQ ID NO:9), linking the intracellular domain of CD3ζ (SEQ ID NO: 5),linking the wild-type IL-15 cytokine (SEQ ID NO: 7) through T2A (SEQ ID NO: 6), a CAR structure targeting BCMA for NK cells was constructed; enzymatic cutting lentivirus vector Pre-Lenti-EF1-CAR V2 and BCMA CAR gene (SEQ ID NO: 13), linking, transformation, clone selection, plasmid extraction, sequencing, and obtaining a sequence-corrected lentiviral vector Pre-Lenti-EF1-CAR V2 NCI-NK.

### 1.2 Preparation of BCMA chimeric antigen receptor lentivirus

1) 293TS cells were cultured in a 37°C, 5% CO2 incubator at 150 rpm. Take the cells to be transfected and count them. The cell density is about 6×106 cells/ml - 7×106 cells/ml, with a cell viability greater than 90% can be used for transfection.
2) Pre-Lenti-EF1-CAR V2 NCI-NK plasmid was mixed with packaging plasmid ZL004:ZL006:envelope plasmid ZL003 in a ratio of 9:7:7:7, and the mixture of the above four plasmids was added to the tube containing 293TS basal medium. Add PEIpro to another tube at a ratio of plasmid:PEIpro=1:2. After incubating the two tubes at room temperature for 5 minutes respectively, slowly add the mixed solution containing PEIpro to the mixture containing DNA, gently shake the centrifuge tube to mix evenly, and incubate at room temperature for about 15 minutes. Add DNA-PEIpro mixed solution to the cells to be transfected, mix evenly, and culture at 37°C and 5% CO2 with shaking. Refill feed after 6 hours. After 48h, collect the culture supernatant containing the lentivirus. Transfer the virus-containing culture supernatant into a 50ml centrifuge tube, centrifuge to remove 293TS cells, filter, concentrate, and package the virus-containing supernatant, store at 80°C for later use.

### Embodiment 2 Preparation of CAR-NK by Cytokine Amplification and Activation

### 1) PBMC Isolation

In a sterile environment, draw 60ml of venous blood from volunteers. Transfer to two 50ml centrifuge tubes, mix, centrifuge for 15 min at 800g, 18-22 °C. Collect the plasma and place in a 50ml centrifuge tube. After removing the plasma, add an equal volume of 0.9% NaCl to dilute the whole blood and mix evenly. Take a new 50ml centrifuge tube, add 15ml of human lymphocyte separation solution respectively, take 20ml of diluted blood and slowly add it onto the separation solution along the side wall of the tube; centrifuge in a horizontal rotor at 400g, 20°C for 30 min (acceleration 3,deceleration 0). Carefully pipette the buffy coat into a new 50ml centrifuge tube, dilute to 40ml with 0.9% NaCl injection solution, and mix evenly by pipetting the centrifuge tube. Centrifuge at 450g and 20°C for 10 min in a horizontal rotor, discard the supernatant, and repeat the wash once. After centrifugation, pour off the supernatant and add 20ml of saline to resuspend and count.

### 2) CD3 T cell depletion

CD3 T cells were depleted with Miltenyi CD3 Regent. Take 6×107 PBMC cells, centrifuge at 300g, 20°C for 10min. Discard the supernatant, add magnetic bead separation solution (80 µl/1×107 cells) to resuspend the cells, add CD3 Reagent (5 µl/1×107 cells), mix well, and place in refrigerator at 2-8°C for 15 min. Add 5ml of magnetic bead separation solution to the cells, mix by pipetting, and centrifuge at 300g, 20°C for 10 min. Install the LS Columns separation column on the magnet and rinse the column with 3ml of magnetic bead separation solution. Discard the supernatant, resuspend the cells with magnetic bead separation solution (100 µl/1×107 cells), mix by pipetting, and pass through the column. Rinse 3ml of magnetic bead separation solution 3 times. Collect the effluent.

### 3) NK cell purification

Sample the effluent after T cell removal and count. Centrifuge at 300g for 10 minutes. Add magnetic bead separation solution (80µl/1×107 cells) to resuspend the cells, add CD56 MicroBeads (20µl/1×107 cells), mix well, and place in refrigerator at 2-8°C for 15 min. Add magnetic bead separation solution to resuspend the cells evenly and centrifuge at 300g for 10 minutes. Install the LS Columns separation column on the magnet and rinse the column with 3ml of magnetic bead separation solution. Discard the supernatant, resuspend the cells with magnetic bead separation solution (500 µl/1×108 cells), mix by pipetting, and pass through the column. Rinse 3ml of magnetic bead separation solution 3 times. Take down the column, add 5ml of magnetic bead separation solution, and transfer to a 15ml centrifuge tube. Centrifuge at 300g for 10 min at 20°C. Discard the supernatant, resuspend the cells in 10ml of 0.9% sodium chloride injection, mix well, count, and centrifuge at 300g, 20°C for 10min. Discard the supernatant and resuspend in NK culture medium (1% of NK MACS Supplement + NK MACS Basal Medium + IL-2(500IU/ml)+IL-15 (140 IU/ml) + 3% autologous plasma). Adjust the cell density to 1×106/ml, and inoculate into a 12-well plate with 1ml/well.

### 4) Lentivirus infection of NK cells

On the third day, observe the NK cells and most of them were found to be aggregated into clumps, mix evenly by pipetting. Add Vectofusin-1wih a final concentration of 10µg/ml, and add virus to MOI = 15; on the 4th day, replenish culture medium; on the 5th day, transfer to a 6-well plate and replenish culture medium; on the 7th day, replenish culture medium; on the 8th day, use target protein combined with flow cytometry to detect the positive rate of CAR expression in BCMA CAR NK cells.

The results are shown in FIG. 2A-2B. FIG. 2A is a negative control, and FIG. 2B is the positive rate of the CAR expression (71%) in BCMA using the BCMA target protein detection.

### Embodiment 3 Preparation of anti-BCMA CAR-NK by amplification and activation of feeder cells

### 1) PBMC Isolation

Collect single blood samples from healthy volunteers. Transfer to a 50ml centrifuge tube and dilute the blood sample with an equal amount of 0.9% NaCl injection solution. Take a new 50ml centrifuge tube, add 15ml of room temperature human lymphocyte separation solution respectively, take 20ml of diluted blood and slowly add it onto the separation solution along the side wall of the tube. Place in a centrifuge, set the centrifugal force to 400g, temperature to 20°C, and centrifuge for 30 minutes (acceleration 3, deceleration 0). Carefully pipette the buffy coat into two other 50ml centrifuge tubes. Add 0.9% sodium chloride injection solution to 40ml and mix evenly by pipetting with pipette. Centrifuge at 400g, 20°C for 10min. Discard the supernatant, add 0.9% sodium chloride injection solution to resuspend the lymphocytes, and replenish injection solution to 40ml. Centrifuge at 400g, 20°C for 10min. After centrifugation, discard the supernatant and add 20ml of 0.9% sodium chloride injection solution. Mix evenly, take a sample and count.

### 2) CD3 T cell depletion

CD3 T cells were depleted with Miltenyi CD3 Regent. Take 1×108 PBMC cells, centrifuge at 300g, 20°C for 10min. Discard the supernatant, add magnetic bead separation solution (80 µl/1×107 cells) to resuspend the cells, add CD3 Reagent (5 µl/1×107 cells), mix well, and place in refrigerator at 2-8°C for 15 min. Add 5ml of magnetic bead separation solution to the cells, mix by pipetting, and centrifuge at 300 g, 20°C for 10 min. Install the LS Columns separation column on the magnet and rinse the column with 3ml of magnetic bead separation solution. Discard the supernatant, resuspend the cells with magnetic bead separation solution (100 µl/1×107 cells), mix by pipetting, and pass through the column. Rinse 3ml of magnetic bead separation solution 3 times. Collect the effluent.

3) NK cell purification: Sample the effluent after T cell removal and count. Centrifuge at 300g for 10 minutes. Add magnetic bead separation solution (80µl/1×107 cells) to resuspend the cells, add CD56 MicroBeads (10µl/1×107 cells), mix well, and place in refrigerator at 2-8°C for 15 min. Add magnetic bead separation solution to resuspend the cells evenly and centrifuge at 300g for 10 minutes. Install the LS Columns separation column on the magnet and rinse the column with 3ml of magnetic bead separation solution. Discard the supernatant, resuspend the cells with magnetic bead separation solution (500 µl/1×108 cells), mix by pipetting, and pass through the column. Rinse 3ml of magnetic bead separation solution 3 times. Take down the column, add 5ml of magnetic bead separation solution, and transfer to a 15ml centrifuge tube. Centrifuge at 300g for 10 min at 20°C. Discard the supernatant, resuspend the cells in 10ml of 0.9% sodium chloride injection, mix well, count, and centrifuge at 300g, 20°C for 10min. Discard the supernatant and resuspend in NK culture medium.

### 4) NK cell activation

1: 1 Mix 7.5×106 NK cells (2.5×105/ml) and 7.5×106 K562-mbIL-21 cells (irradiated, 100 Gy) (2.5×105/ml) evenly with 30ml NK cell culture medium in T-75 bottle. Place in a 37°C, 5% CO2 incubator for incubation. Observe the cells on day 2, day 3, and day 4. On the 5th day, the medium was changed by centrifugation, and the cells were transferred to a 50ml centrifuge tube. The cells were mixed and counted, and centrifuged at 300g, 20°C for 10min. Discard the supernatant and resuspend NK cell culture medium (1% of NKMACS Supplement + NK MACS Basal Medium + IL-2 (200 IU/ml) + 5% autologous plasma). The cell density was adjusted to 1×106 /ml.

### 5) Lentivirus infection of NK cells

On the 6th day, observe NK cells and found that most of them were fully activated and proliferated vigorously. Retronectin coated plates, inoculate with cells, add Vectofusin-1 with a final concentration of 10 µg/ml, add virus to MOI = 10.

On day 7, replenish the culture medium.

On day 8, ativate CAR NK cells again through mix CAR NK cells and K562-mbIL-21 cells in a 1:1 ratio evenly. Place in a 37°C, 5% CO2 incubator for incubation.

Observe the cells on day 9, day 10.

On the 11th day, the medium was changed by centrifugation, and the cells were transferred to a 50ml centrifuge tube. The cells were mixed and counted, and centrifuged at 300 g, 20°C for 10min. Discard the supernatant and resuspend NK cell culture medium (1% of NK MACS Supplement + NK MACS Basal Medium + IL-2 (200 IU/ml) + 3% autologous plasma). The cell density was adjusted to 1×106 /ml.

Observe the cells on day 12.

On day 13, replenish fluid, adjust the cell density to 1×106/ml.

On day 14, detect the CAR positivity rate of BCMA CAR NK cells using target protein combined with flow cytometry.

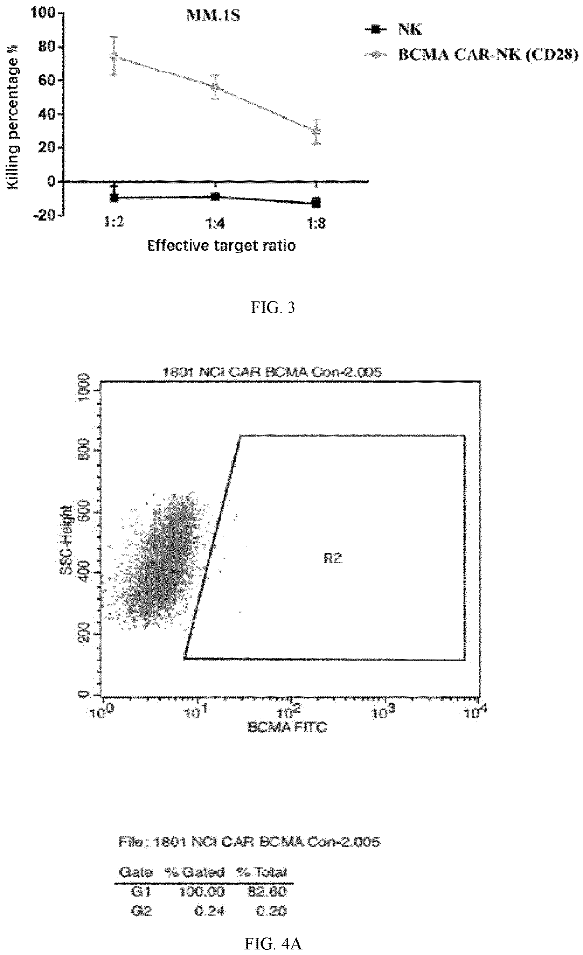

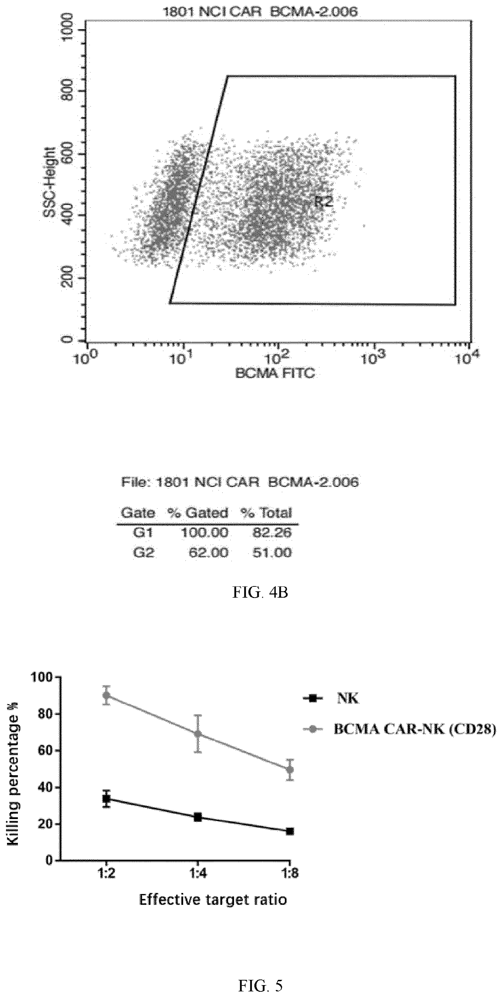

The results are shown in FIG. 4A-4B. FIG. 4A is a negative control, and FIG. 4B is the positive rate of the CAR expression (62%) in BCMA using the BCMA target protein detection.

### Embodiment 4 Evaluation of Function of anti-BCMA CAR-NK cells

In vitro cell killing experiments were performed using the LDH detection kit (Promega). Four gradients were set for anti-BCMA CAR-NK cells and target cells (MM.1S cells with high expression of BCMA molecules, purchased from ATCC) prepared in Embodiment 2 or 3 according to their number ratio (i.e. effective target ratio) of 1:2, 1:4, 1:8 and 1:16. In particular, the target cells were 3×104 cells/well, and the system of all remaining wells were supplemented to 200 µL with XVIVO contained medium/1640 culture medium. Place the 96-well plate in a 37°C, 5% CO2 incubator for incubation. After 17 hours, 20µL of lysis solution was added to the well with maximum release, mixed to completely rupture the cells, and the 96-well plate was placed in a CO2 incubator and incubated for 2 hours. After 2 hours, observe the well with the maximum release. After all the target cells were lysed, suck 50 µL of supernatant from each well into a flat-bottom 96-well plate, and then add 50 µL of substrate solution to each well to develop color in the dark for 30min. After 30 min, observe the color change, in which the well with the maximum release of MM.1S and the well containing anti-BCMA CAR-NK cells were darker. The measurement was performed using an enzyme marker at a wavelength of 490 nm. The results of the lethality test were obtained using the LDH detection kit.

The killing effect of anti-BCMA CAR-NK (CD28 hinge) cells prepared in Embodiment 2 on MM.1S cells is shown in FIG. 3. The results show that the anti-BCMA CAR-NK cells prepared in Embodiment 2 can specifically kill BCMA positive cells, and the killing activity is very high, all above 20%.

The killing effect of anti-BCMA CAR-NK (CD28 hinge) cells prepared in Embodiment 3 on MM.1S cells is shown in FIG. 5. The results show that the anti-BCMA CAR-NK cells prepared in Embodiment 3 can specifically kill BCMA positive cells, and the killing activity is very high, all above 40%.

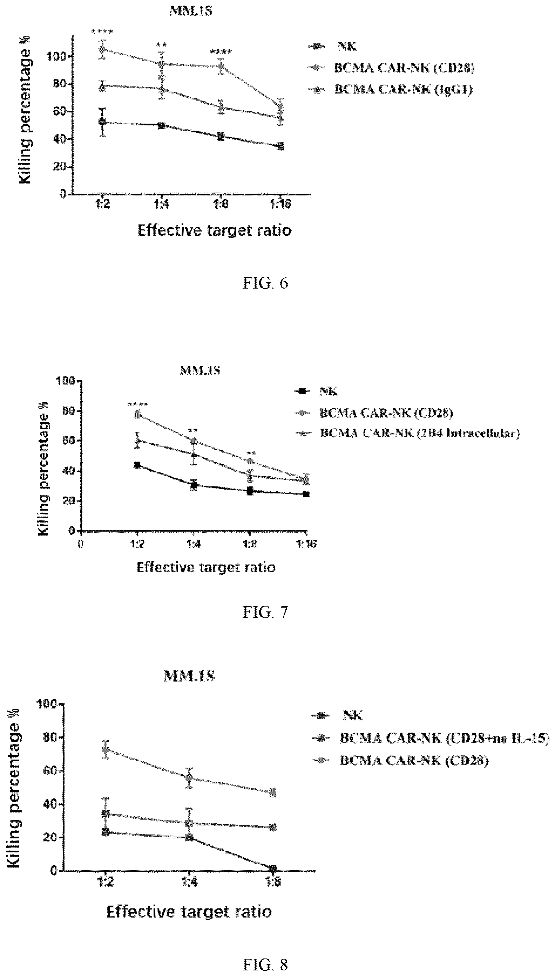

The killing effect on MM.1S cells of anti-BCMA CAR-NK (CD28 hinge, CAR sequence shown in SEQ ID NO:13) cells, anti-BCMA CAR-NK (IgG1 hinge, CAR sequence shown in SEQ ID NO:14) cells, anti-BCMA CAR-NK (2B4 Intracellular, CAR sequence is shown in SEQ ID NO:15), anti-BCMA CAR-NK (CD28 hinge + no IL-15, CAR sequence is shown in SEQ ID NO:12) prepared in Embodiment 3 is refer to FIG.6-8. The results showed that the killing effect in vitro of anti-BCMA CAR-NK cells with CD28 extracellular region as hinge region was better than that of anti-BCMA CAR-NK cells with IgG1 molecular hinge region as hinge region (FIG. 6); the killing effect in vitro of anti-BCMA CAR-NK cells with CD28 cytoplasm is better than that of anti-BCMA CAR-NK cells with 2B4 cytoplasm (FIG. 7); the killing effect in vitro of anti-BCMA CAR-NK cells secreting IL-15 is better than that of anti-BCMA CAR-NK cells expressing CAR molecules but not secreting IL-15 (FIG. 8).

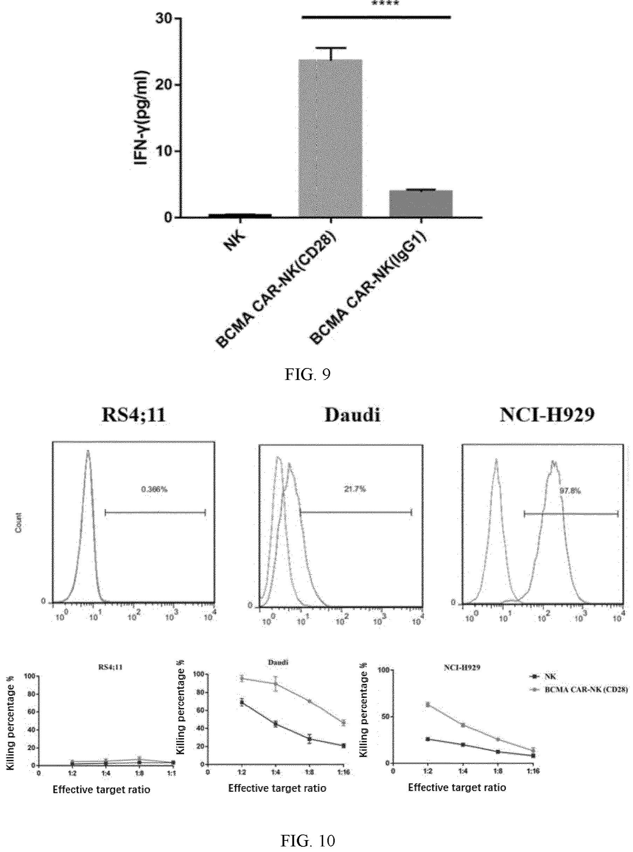

After killing, collect the supernatant and determine the level of cytokine IFN-γ, the results are as shown in FIG. 9, compared with the anti-BCMA CAR-NK cells with the hinge region of IgG1 molecule hinge region, the anti-BCMA CAR-NK cells with the hinge region of CD28 extracellular region can significantly up-regulate the expression of cytokine IFN-γ with tumor killing effect in vitro, which verifies the specific killing effect of anti-BCMA CAR-NK (CD28 hinge) cells.

### Embodiment 5:

In vitro cell killing experiments were performed using the LDH detection kit (Promega). Four gradients were set for anti-BCMA CAR-NK cells and target cells (RS4;11 without BCMA expression, Daudi with BCMA expression and NCI-1299 tumor cells) prepared in Embodiment 3 according to their number ratio (i.e. effective target ratio) of 1:2, 1:4, 1:8 and 1:16. In particular, the target cells were 3×104 cells/well, and the system of all remaining wells were supplemented to 200 µL with X-VIVO contained medium/1640 culture medium. Place the 96-well plate in a 37°C, 5% CO2 incubator for incubation. After 17 hours, 20µL of lysis solution was added to the well with maximum release, mixed to completely rupture the cells, and the 96-well plate was placed in a CO2 incubator and incubated for 2 hours. After 2 hours, observe the well with the maximum release. After all the target cells were lysed, suck 50 µL of supernatant from each well into a flat-bottom 96-well plate, and then add 50 µL of substrate solution to each well to develop color in the dark for 30min. After 30 min, observe the color change, in which the well with the maximum release and the well containing anti-BCMA CAR-NK cells were darker. The measurement was performed using an enzyme marker at a wavelength of 490 nm. The results of the lethality test were obtained using the LDH detection kit.

FIG. 10 shows that anti-BCMA CAR-NK (CD28 hinge) cells kill tumor cells with different levels of BCMA expression in vitro. The results showed that there was no killing effect on RS4;11 which did not express BCMA, there was killing effect on Daudi and NCI-1299 tumor cells which expressed BCMA. The anti-BCMA CAR-NK (CD28 hinge) cells in this application have BCMA specificity.

### Embodiment 6:

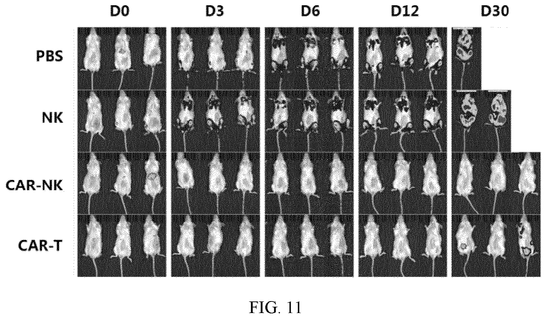

The human multiple myeloma MM.1S cell line was transduced to express the luciferase reporter gene (luciferase) to obtain MM.1S-luc. Use 1640 culture medium containing 10% fetal bovine serum (FBS), place in an incubator with 5% carbon dioxide and 37°C for routine culture. MM.1S-luc cell line was injected into the tail vein of NSG mice (50 mice, half male and half female) at 1.5×106 cells/200µl PBS. After 17 days, all animals were imaged. Tumor-bearing mice entered the experimental group and were randomly divided into 5 groups (half male and half female, 8 mice in each group), which were used as PBS group, NK cell group, anti-BCMA CAR-NK (CD28 hinge) cell group, BCMA CAR-T (CD28 hinge) cell group, respectively. In particular, PBS were injected into the tail vein of the extracellular fluid group, NK cells were injected into the tail vein of the NK cell group, anti-BCMA CAR-NK (CD28 hinge) cells prepared in Embodiment 3 were injected into the tail vein of the anti-BCMA CAR-NK (CD28 hinge) cells group, and BCMA CAR-T(CD28 hinge) cells were injected into the tail vein of BCMA CAR-T(CD28 hinge) cells group. 3 days later, anesthetize the mice, intraperitoneally inject Luciferase substrate. Observe the tumor burden in the control group and the treatment group using a small animal in vivo imaging system. The results on the 3rd, 6th, 12th and 30th days after treatment are shown in FIG.11, respectively.

The results showed that there was almost no tumor cell imaging in the anti-BCMA CAR-NK cell treatment group, while the tumor burden of the control group mice was severe, which indicates that anti-BCMA CAR-NK cells can effectively eliminate BCMA-positive tumor cells. After intravenous reinfusion of Anti-BCMA CAR-NK cells, the fluorescence signal intensity in the mice began to decrease on the third day compared with the PBS group and NK cell group, which indicates that the tumor burden was decreasing. As shown in FIG. 11, the fluorescence signal intensity in mice on day 6 was basically undetectable, which indicates that anti-BCMA CAR-NK also showed a strong therapeutic effect against BCMA expression-related diseases in animal experiments.

The above description is only a preferred embodiment of the present application and is not intended to limit the present application. Any modifications, equivalent substitutions, improvements, etc. made within the spirit and principle of the present application should be included in the protection scope of the present application.

## Claims

1. A fusion polypeptide, **characterized in that**, comprises a chimeric antigen receptor and an enhancer capable of enhancing one or more activities of immune effector cells, wherein the chimeric antigen receptor comprises a BCMA binding domain and a non-antigen binding domain, wherein the non-antigen binding domain comprises an extracellular domain, a transmembrane domain and an intracellular co-stimulatory domain of CD28 or a functional variant thereof, and a signaling domain of CD3ζ or a functional variant thereof; and the enhancer is selected from IL-2, IL-3, IL-4, IL-6, IL-7, IL-8, IL-10, IL-11, IL-12, IL-15, IL-17, IL-18, IL-21, IL-23, one or more of receptors or functional variants thereof.

2. The fusion polypeptide according to claim 1, **characterized in that**, the BCMA binding domain comprises heavy chain complementary determining region 1, heavy chain complementary determining region 2 and heavy chain complementary determining region 3, wherein the heavy chain complementary determining region 1 comprises an amino acid sequence shown in SEQ ID NO: 2, the heavy chain complementary determining region 2 comprises an amino acid sequence shown in SEQ ID NO: 3, and the heavy chain complementary determining region 3 comprises an amino acid sequence shown in SEQ ID NO: 4.

3. The fusion polypeptide according to claim 2, **characterized in that**, the BCMA binding domain comprises a single domain antibody.

4. The fusion polypeptide according to claim 3, **characterized in that**, the BCMA binding domain comprises an amino acid sequence shown in SEQ ID NO: 8 or a functional variant thereof.

5. The fusion polypeptide according to claim 1, **characterized in that**, the extracellular domain, transmembrane domain and intracellular co-stimulatory domain of CD28 comprise an amino acid sequence shown in SEQ ID NO:9.

6. The fusion polypeptide according to claim 1, **characterized in that**, the signal transduction domain of CD3ζ comprises an amino acid sequence shown in SEQ ID NO:5.

7. The fusion polypeptide according to claim 1, **characterized in that**, the non-antigen binding domain comprises an amino acid sequence shown in SEQ ID NO:10.

8. The fusion polypeptide according to claim 1, **characterized in that**, the chimeric antigen receptor further comprises a signal peptide fragment, wherein a C-terminus of the signal peptide fragment is linked to a N-terminus of the antigen binding domain; the signal peptide fragment comprises an amino acid sequence shown in SEQ ID NO: 1.

9. The fusion polypeptide according to claim 8, **characterized in that**, the chimeric antigen receptor comprises an amino acid sequence shown in SEQ ID NO: 11 or an amino acid sequence shown in SEQ ID NO: 12.

10. The fusion polypeptide according to claim 1, **characterized in that**, the enhancer comprises IL-15 or its functional variant, or comprises a chimeric cytokine receptor of IL-15 receptor.

11. The fusion polypeptide according to claim 10, **characterized in that**, the enhancer comprises an amino acid sequence shown in SEQ ID NO:7.

12. The fusion polypeptide according to claim 1, **characterized in that**, the fusion polypeptide comprises an amino acid sequence shown in SEQ ID NO: 13.

13. An isolated nucleic acid molecule, **characterized in that**, encoding the fusion polypeptide according to any one of claims 1-12.

14. One or more isolated nucleic acid molecules, **characterized in that**, comprising a nucleotide sequence encoding a chimeric antigen receptor, wherein the chimeric antigen receptor comprises a BCMA binding domain and a non-antigen binding domain, wherein the non-antigen binding domain comprises an extracellular domain, a transmembrane domain and an intracellular co-stimulatory domain of CD28 or a functional variant thereof, and a signaling domain of CD3ζ or a functional variant thereof; and a nucleotide sequence encoding an enhancer capable of enhancing one or more activities of immune effector cells.

15. The one or more isolated nucleic acid molecules according to claim 14, **characterized in that**, the nucleotide sequence encoding the chimeric antigen receptor comprises a nucleotide sequence shown in SEQ ID NO:22.

16. The one or more isolated nucleic acid molecules according to claim 14, **characterized in that**, the nucleotide sequence encoding the enhancer is shown in SEQ ID NO: 20.

17. The one or more isolated nucleic acid molecules according to claim 14, **characterized in that**, comprising a nucleotide sequence shown in SEQ ID NO:23.

18. One or more isolated constructs, **characterized in that**, comprising the nucleic acid molecule according to claim 13 or the one or more isolated nucleic acid molecules according to any one of claims 14-17.

19. The one or more isolated constructs according to claim 18, **characterized in that**, comprising a first nucleic acid molecule and a second nucleic acid molecule, wherein the first nucleic acid molecule comprises a nucleotide sequence encoding a chimeric antigen receptor, wherein the chimeric antigen receptor comprises a BCMA binding domain and a non-antigen binding domain, wherein the non-antigen binding domain comprises an extracellular domain, a transmembrane domain and an intracellular co-stimulatory domain of CD28 or a functional variant thereof, and a signaling domain of CD3ζ or a functional variant thereof; the second nucleic acid molecule comprises a nucleotide sequence encoding an enhancer capable of enhancing one or more activities of immune effector cells.

20. A cell, **characterized in that**, comprising the nucleic acid molecule according to claim 13, or the one or more isolated nucleic acid molecules according to any one of claims 14-17, or the one or more isolated constructs according to any one of claims 18-19, and/or expressing the fusion polypeptide according to any one of claims 1-12.

21. The cell according to claim 20, **characterized in that**, the cell comprises immune effector cells.

22. The cell according to claim 21, **characterized in that**, the immune effector cell comprises T cells, B cells, natural killer cells, macrophages, NKT cells, monocytes, dendritic cells, granulocytes, lymphocytes, leukocytes and/or peripheral blood mononuclear cells.

23. The cell according to claim 22, **characterized in that**, comprising engineered immune effector cells.

24. The cell according to claim 23, **characterized in that**, the engineered immune effector cells comprise CAR-T cells or CAR-NK cells.

25. A method for modifying cells, **characterized in that**, the method comprises introducing a first nucleic acid molecule and a second nucleic acid molecule into the cell, wherein the first nucleic acid molecule encodes a chimeric antigen receptor, the chimeric antigen receptor comprises a BCMA binding domain and a non-antigen binding domain, wherein the non-antigen binding domain comprises an extracellular domain, a transmembrane domain and an intracellular co-stimulatory domain of CD28 or a functional variant thereof, and a signaling domain of CD3ζ or a functional variant thereof; and the second nucleic acid molecule encodes a nucleotide sequence of an enhancer capable of enhancing one or more activities of the engineered immune effector cells.

26. A pharmaceutical composition, **characterized in that**, comprising the nucleic acid molecule according to claim 13, or the one or more isolated nucleic acid molecules according to any one of claims 14-17, the one or more isolated constructs according to any one of claims 18-19, or the cell according to any one of claims 20-25, and optionally a pharmaceutically acceptable carrier.

27. An use of the fusion polypeptide according to any one of claims 1-12, the nucleic acid molecule according to claim 13, or the one or more isolated nucleic acid molecules according to any one of claims 14-17, the one or more isolated constructs according to any one of claims 18-19, or the cell according to any one of claims 20-25 for preparing a drug, **characterized in that**, the drug is used to treat tumors.
